# EUROPEAN PATENT APPLICATION

(11) **EP 4 710 991 A1**
(43) Date of publication of application: **18.03.2026**
(21) Application number: 24200926.4
(22) Date of filing: 17.09.2024
(51) Int. Cl.: A61M 60/13, A61M 60/174, A61M 60/178, A61M 60/237, A61M 60/31, A61M 60/414, A61M 60/531, A61M 60/569, A61M 60/806, A61M 60/812, A61M 60/861, A61M 60/873, A61M 60/876

(54) **A DRIVE TRANSFER SYSTEM TO DRIVE A REMOTE IMPLANTABLE MEDICAL DEVICE**

(71) Applicant: Pumpinheart Limited, Blackrock, Co. Dublin A94 W3V1 (IE)
(72) Inventor: ENNETT, Garry, Galway, H91 DCH9 (IE); COLGAN, Darragh, Galway, H91 DCH9 (IE); MALONE, Andrew, Galway, H91 DCH9 (IE); HAMEED, Aamir, Galway, H91 DCH9 (IE); HICKEY, Donald, Galway, H91 DCH9 (IE)
(74) Representative: Purdylucey Intellectual Property

(57) **Abstract**

A drive transfer system (40) configured to drive a remote implantable medical device (1) configured to treat disease, the system (40) comprising a motor (42) configured to actuate an implantable medical device (1), a controller device (41) communicable with the motor (42) configured to modify the output parameters of the motor (42) in accordance with the treatment required, and a drive shaft (8) configured to operably connect the motor (42) to the implantable medical device (1) to drive the implantable medical device (1) when the motor (42) is located remotely from the implantable medical device (1). The system of the invention finds particular utility in the treatment of cardiac diseases such as heart failure with preserved ejection fraction (HFpEF).

## Description

### Field of the Invention

The present invention relates to a drive transfer system. More particularly, the invention relates to a drive transfer system configured to drive a remote implantable medical device configured to treat disease. The invention also relates to a system to treat cardiac disease such as heart failure with preserved ejection fraction (HFpEF) or heart failure with reduced ejection fraction (HFrEF). The invention also relates to a method of treating heart disease such as heart failure with preserved ejection fraction (HFpEF) or heart failure with reduced ejection fraction (HFrEF), and to a method of relieving or preventing secondary pulmonary hypertension.

### Background to the Invention

Implantable mechanical medical devices such as pumps are widely used in medicine to deliver drugs and the like to patients and in coronary care to improve heart performance. In general, such devices form part of a system provided with a motor at the implantable medical device and a power source connected to the motor via a lead and located remote from the medical device. The power source, together with a controller, can also be implanted if desired e.g. beneath the clavicle in systems to treat heart failure.

Cardiac disease such as heart failure (HF) is one of the many diseases in which implantable medical devices are employed to treat the dysfunction. HF is defined as inability of the heart to supply adequate blood to the body. As the demographics suggest, with increasing ageing population, prevalence of heart failure is also increasing. The cardinal manifestations of HF are dyspnoea and fatigue, which may limit exercise tolerance, and fluid retention, which may lead to pulmonary and/ or splanchnic congestion and/or peripheral oedema.

The heart cycle has two phases; a contraction phase when heart pumps the blood to the whole body and the relaxation phase when heart is filled with blood. Heart failure associated with the contraction phase (systole) includes HF with reduced ejection fraction (HFrEF), a condition commonly caused by ischemic heart disease that leads to decrease in stroke volume and cardiac output which results in the activation of neurohormonal response in order to restore the normal cardiac output. Left ventricular assist devices have been developed to assist the heart during the contraction phase, including pump devices designed to assist the pumping of blood into the aorta during diastole (contraction), and contractile devices designed to be implanted between two walls of the left ventricle (LV) that reciprocate in a pattern synergistic with the heart rhythm to assist the contraction of the left ventricle during systole. Pump devices for treatment of HFrEF are described in US2016/000983 and US7942804.

Heart disease associated with the relaxation phase of the heart cycle (diastole) includes heart failure with preserved ejection fraction (HFpEF). HFpEF is a clinical syndrome in which patients have symptoms and signs of HF with normal or near normal left ventricular ejection fraction (LVEF >50 percent). During early diastole phase of the cardiac cycle, the healthy LV acts as a vacuum cleaner' that enhances the suction particularly during exercise. In HFpEF, cardiomyocyte stiffness results in the loss of this relaxation enhancement, hence normal LV filling is dependent on high left atrial (LA) pressure to push blood into the LV. This pressure elevation can further cause atrial remodelling and secondary pulmonary hypertension, predisposing patients to develop atrial fibrillation and right ventricular (RV) dysfunction. Each 10 mm Hg increment in pulmonary artery pressure in patients with HFpEF was found to be associated with a 28% increase in 3-year mortality 3. Hence, there is a need to effectively control the death rate from HFpEF.

A disadvantage of known mechanical implantable medical devices for the treatment of cardiac disease where the motor is implanted with the medical device is that the size of the medical device is increased which can hinder implantation via the vasculature. In addition, the motor can generate heat giving rise to potential patient tissue damage. This is particularly the case where the motor is located in or adjacent critical tissue such as heart tissue. Furthermore, over the lifetime of the product, the replacement of motors which are directly coupled to and implanted with implanted medical device can be complex resulting in the need for repeated invasive procedures. Similar disadvantages arise where implantable medical devices are employed for the treatment of non-cardiac diseases.

An objective of the invention to overcome at least one of the above-referenced problems.

### Summary of the Invention

The objective of the invention is met by the provision of a remote drive transfer system and method to treat disease that employs an implantable mechanical medical device connected to a remote motor, separate from the implantable medical device, via a drive shaft, preferably a flexible drive shaft, which can transmit torque, translational movement, rotational velocity or a combination thereof to the implantable medical device from the motor to drive the implantable medical device even when the motor is located remotely from the implantable medical device. The implantable medical device can be any powered device for the treatment of disease such as an impeller-type of device for the treatment of cardiac dysfunction such as HFpEF, HFrEF or other conditions. In one specific form, the invention provides a system and method for the treatment of cardiac dysfunction e.g. HFpEF that employs a blood pumping device implanted in the left ventricle configured for sequential activation during diastole (to assist the left ventricle pull blood into the left ventricle from the left atrium through the open mitral valve) and deactivation during systole when the left ventricle is emptying. This is made possible by implanting the blood pumping device in the left ventricle. A controller device is operatively coupled to the blood pumping device and configured to remotely activate and deactivate the blood pumping device via the (flexible) shaft in a pattern synergistic with the cardiac cycle of the subject comprising activation during ventricular diastole and deactivation during ventricular systole. The controller device may receive signals from a sensor, which may form part of the blood pumping device or may be a separate sensor such as an ECG or atrial sensor. The blood pumping device generally comprises an impeller and ideally is an axial flow pump. In use, the blood pumping device may be anchored to a wall of the left ventricle and positioned such that upon activation the pressure in the top of the left ventricle adjacent the mitral valve is reduced sufficiently to assist the drawing of blood into the left ventricle from the right atrium.

In a first aspect, the invention provides a drive transfer system configured to drive a remote implantable medical device configured to treat disease, the system comprising:
a motor configured to actuate an implantable medical device;
a controller device communicable with the motor configured to modify the output parameters of the motor in accordance with the treatment required, and
a drive shaft configured to operably connect the motor to the implantable medical device to drive the implantable medical device when the motor is located remotely from the implantable medical device.

In any embodiment, the drive transferred by the drive shaft from the motor to the implantable medical device is torque, rotary motion, translational movement or any combination thereof.

In any embodiment, the drive shaft comprises a flexible shaft.

In any embodiment, the flexible shaft comprises a flexible cable.

In any embodiment, the flexible shaft is surrounded by a catheter.

In any embodiment, the motor is optionally integral with the controller device and the flexible shaft is configured to attach to and detach from the motor or the controller device.

In any embodiment, the controller device comprises an implantable controller device.

In any embodiment, the controller device is configured to control the amount of torque and rotational velocity required to drive the implantable medical device.

In any embodiment, the system further comprises a power unit to power the motor.

In any embodiment, the system further comprises an implantable medical device.

In any embodiment, the power unit is chargeable, optionally wirelessly chargeable.

In any embodiment, the implantable medical device comprises a pump and/or an impeller.

In any embodiment, the controller device is configured to modify the output parameters of the implantable medical device or the motor so as to activate and deactivate implantable medical device or the motor.

In any embodiment, the disease is any cardiac dysfunction or disease such as heart failure with preserved ejection fraction (HFpEF) or heart failure with reduced ejection fraction (HFrEF)and the implantable medical device comprises a blood pumping device.

In any embodiment, the disease is HFpEF and blood pumping device is configured for implantation in a left ventricle of a heart of a subject, in which the blood pumping device is configured to draw blood from a left atrium into the left ventricle of the heart through a mitral valve upon activation.

In any embodiment, the system further comprises an anchoring assembly for anchoring the blood pumping device to the heart.

In any embodiment, the controller device is configured to modify the output parameters of the blood pumping device to activate and deactivate the blood pumping device in a pattern synergistic with a cardiac cycle of the subject comprising activation during diastole and deactivation during systole.

In any embodiment, the system further comprises at least one sensor in communication with the controller device to detect one or more parameters associated with the heart, wherein the controller device is configured to modify the output parameters of the pump or impeller based on the one or more detected parameters received from the at least one sensor.

In any embodiment, there is at least one sensor is configured to detect one or more parameters selected from heart rate, closure of the aortic valve, opening of the aortic valve, closure of the mitral valve, and opening of the mitral valve and the controller is configured to adjust the frequency of activation and deactivation of the pump or impeller based on the one or more parameters sensed by the sensor.

In any embodiment, the at least one sensor is configured to detect a blood pressure parameter of the subject's heart and the controller is configured to adjust the pump flow rate of the blood pumping device to correspond to the blood pressure parameter sensed by the sensor.

In any embodiment, the pump is a blood pumping device comprising:
an external tubular housing;
a blood inlet in the tubular housing,
an oppositely disposed blood outlet in the tubular housing;
an internal lumen extending between the blood inlet and blood outlet, and
an impeller mounted on a rotor within the housing to pump blood from the blood inlet to the blood outlet, wherein the drive shaft is operably connected to the rotor to rotate the rotor during activation of the blood pumping device.

In another embodiment, the invention provides a system to treat cardiac disease such as HFpEF or HFrEF comprising:
an implantable device configured for implantation in the heart of a subject;
a motor configured to mechanically power the implantable medical device;
a controller device communicable with the motor configured to modify the output parameters of the motor in accordance with the treatment required, and
a drive shaft configured to operably connect the motor to the implantable medical device to drive the blood pumping device when the motor is located remotely from the blood pumping device.

In any embodiment, the drive transferred by the drive shaft from the motor to the implantable medical device is torque, rotary motion, translational movement or any combination thereof.

In one embodiment, the drive shaft is a flexible shaft.

In one embodiment, the invention provides a system to treat heart failure with preserved ejection fraction (HFpEF) comprising:
a blood pumping device configured for implantation in a left ventricle of a heart of a subject;
a motor configured to mechanically actuate the blood pumping device;
a controller device communicable with the motor configured to modify the output parameters of the motor in accordance with the treatment required, and
a shaft configured to operably connect the motor to the blood pumping device to drive the blood pumping device when the motor is located remotely from the blood pumping device.

In any embodiment, the drive transferred by the drive shaft from the motor to the implantable medical device is torque, rotary motion, translational movement or any combination thereof.

In one embodiment, the drive shaft is a flexible shaft.

In any embodiment, the flexible shaft comprises a flexible cable.

In any embodiment, the flexible shaft is surrounded by a catheter.

In any embodiment, the motor is optionally integral with the controller device and the flexible shaft is configured to attach to and detach from the motor or the controller device.

In any embodiment, the controller device comprises an implantable controller device.

In any embodiment, the controller device is configured to control the amount of drive required to drive the implantable medical device.

In any embodiment, the system further comprises a power unit to power the motor. In any embodiment, the power unit is chargeable, optionally wirelessly chargeable.

In any embodiment, the system is to treat, relieve or prevent secondary pulmonary hypertension.

In any embodiment, the system is to treat a condition associated with impaired filling of the left ventricle during ventricular diastole.

In any embodiment, the system further comprises at least one sensor in communication with the controller device to detect one or more parameters associated with the heart, wherein the controller device is configured to modify the output parameters of the blood pumping device based on the one or more detected parameters received from the at least one sensor.

In any embodiment, the at least one sensor is configured to detect one or more parameters selected from heart rate, closure of the aortic valve, opening of the aortic valve, closure of the mitral valve, and opening of the mitral valve and the controller device is configured to adjust the frequency of activation and deactivation of the blood pumping device based on the one or more parameters sensed by the sensor.

In any embodiment, the at least one sensor is configured to detect a blood pressure parameter of the subject's heart and the controller device is configured to adjust the pump flow rate of the blood pumping device to correspond to the blood pressure parameter sensed by the sensor.

In any embodiment, the controller device is configured to modify the pump flow rate and/or the frequency of actuation of motor and/or the blood pumping device.

In any embodiment, the controller device is configured to modify the amplitude of the voltage of the power supply to the motor. When the pump comprises an impeller, varying the amplitude of the voltage modifies the speed of rotation of the impeller.

In any embodiment, the controller device is configured to modify the frequency of the voltage duty cycle of the power supply to the motor. Varying the frequency of the voltage controls the activation and deactivation of the pump.

In any embodiment, the at least one sensor is selected from the group consisting of: a pressure sensor, a wireless pressure sensor, a mems pressure sensor, an artery pressure sensor, a cardiac output (CO) sensor, a blood pressure sensor, an ejection fraction of the left ventricle, a heart rate sensor, a motion sensor, an accelerometer, an ECG (Electrocardiogram) sensor, an O2 saturation sensor, a micro accelerometer, and a sonomicrometer. The pressure sensor can be a ventricular and/or atrial pressure sensor.

In any embodiment, the sensor is a heart pacing sensor.

In any embodiment, the sensor is an atrial or ventricular pressure sensor.

In any embodiment, the system comprises a heart pacing sensor and an atrial or ventricular pressure sensor.

In any embodiment, the sensor is integral with the blood pumping device.

In any embodiment, the sensor is coupled to heart tissue.

In any embodiment, the blood pumping device comprises an impeller.

In any embodiment, the blood pumping device comprises an axial flow pump.

In any embodiment, the blood pumping device comprises:
an external tubular housing;
a blood inlet in the tubular housing,
an oppositely disposed blood outlet in the tubular housing;
an internal lumen extending between the blood inlet and blood outlet, and
an impeller mounted on a rotor within the housing to pump blood from the blood inlet to the blood outlet, wherein the flexible drive shaft is operably connected to the rotor to rotate the rotor during activation of the blood pumping device.

In any embodiment, the fluid inlet of the housing extends into or towards the left atrium through the mitral valve.

In any embodiment, the blood pumping device is configured to be completely contained within the left ventricle with only part of the fluid inlet extending into the left atrium. Alternatively, the blood pumping device can extend between the left ventricle and the left atrium.

In any embodiment, the blood pumping device comprises a fluidic extension conduit configured to provide fluidic communication from inside the left atrium to the fluid inlet of the housing.

In any embodiment, the fluidic extension conduit is detachably coupled to the fluid inlet of the housing.

In any embodiment, the fluidic extension conduit is flexible.

In any embodiment, the impeller is disposed on the rotor at a fluid outlet side of the housing.

In any embodiment, the tubular housing comprises a first cylindrical part connected to a second cylindrical part by a plurality of struts.

In any embodiment, the inlet and outlet are aligned along a common axis.

In any embodiment, the blood pumping device comprises:
a first cylindrical part comprising the motor;
a second cylindrical part comprising the impeller;
a central part connecting the first cylindrical part and second cylindrical part comprising the fluid inlet; and
a fluid outlet disposed in the first cylindrical part or second cylindrical part,
wherein the rotor extends from the motor to the impeller through the central part.

In any embodiment, the fluid inlet comprises one or more apertures in the central part of the housing.

In any embodiment, the second part comprises the fluid outlet. In this embodiment, rotation of the impeller pulls blood through the fluid inlet and through the second part and out of the fluid outlet.

In any embodiment, the second part of the housing has a diameter D1 that is greater than a diameter D2 of the first part of the housing. In any embodiment, a ratio of D1 to D2 is in a range of 3:2 to 5:2.

In any embodiment, the central part of the hosing has a frustoconical shape.

In any embodiment, the central part of the housing comprises a plurality of struts connecting the first and second parts of the housing.

In any embodiment, the fluid outlet is disposed in the first part. In this embodiment, the first part has a lumen for fluid to allow the fluid to pass the motor and exit through the fluid outlet.

In any embodiment, the motor is an electromagnetic motor.

In any embodiment, the impeller comprises an axial hub and at least two vanes mounted to the hub, in which each vane has an elongated swept profile.

In any embodiment, each vane has a hub to tip ratio (v) of 0.20 to 0.30.

In any embodiment, the impeller comprises two vanes disposed on opposed sides of the hub.

In any embodiment, each vane has an axial length of 5 to 20 to mm, preferably 10 to 15 mm, and ideally about 12 to about 13 mm.

In any embodiment, each vane has a radial width of 1 to 10 mm, 3 to 7 mm, 4 to 6 mm, or ideally about 5 mm.

In any embodiment, each vane extends around the hub by a sweep angle of 70° to 140°, 80° to 120°, 90° to 120°, or about 100° to about 120°. The sweep angle refers to the angle that the vane sweeps around the hub.

In any embodiment, a clearance between the radial tip of each vane and the hub housing is 0.1 mm to 1.5 mm, 0.3 mm to 0.7 mm, 0.4 mm to 0.6 mm, and ideally about 0.5 mm.

In any embodiment, the controller device is configured to receive heart rate data from a sensor and modify the output parameters of the blood pumping device in real time based on the received heart rate data.

In any embodiment, the controller is configured to compare the heart rate data with reference impeller rotational speed data, calculate an impeller rotational speed based on the comparison, and actuate the blood pumping device during diastole phases to rotate the impeller at the calculated impeller rotational speed.

In any embodiment, the blood pumping device is dimensioned for percutaneous delivery to the left ventricle of the heart inside a delivery catheter of up to 32 Fr, typically along a guidewire.

In any embodiment, the blood pumping device is configured for mounting on a guidewire.

In any embodiment, the optional anchoring assembly comprises one or more anchor elements (e.g., arms) coupled to the blood pumping device.

In any embodiment, the optional anchoring assembly is configured for mounting to a wall, septum or apex of the left ventricle or left atrium of the heart, to the pulmonary artery, parts of the mitral valve, or a combination of one or more of these.

In any embodiment, the anchoring assembly comprises a plurality of anchoring arms configured for adjustment from a stowed position suitable for percutaneous delivery to a deployed position in which the plurality of anchoring arms oppose the heart.

In any embodiment, the anchoring arms are attached to a distal end of the housing.

In any embodiment, the anchoring arms are configured for self-deployment. For example, each arm may be biased into a deployed position, and self-deploy when the device is advanced beyond a distal end of a delivery sheath.

In any embodiment, the anchoring assembly is configured to couple to the blood pumping device in-vivo.

In any embodiment, the anchoring assembly comprises an anchoring hub configured for coupling to the blood pumping device and a plurality of anchoring arms extending from the hub.

In any embodiment, the system further comprises electronic circuitry for setting the output parameters of the motor, wherein the electronic circuitry is coupled between the controller device and the blood pumping device, and wherein the controller device is configured to send control signals to the electronic circuitry to modify the output parameters of the blood pumping device.

In any embodiment, the controller device is configured for implantation.

In any embodiment, the controller is configured for implantation under the skin of a subject's chest, typically sub dermally.

In any embodiment, the system further comprises a power unit associated with the blood pumping device.

In any embodiment, the power unit is associated with the electronic circuitry.

In any embodiment, the power unit is configured for implantation.

In any embodiment, the power unit is configured for implantation under the skin of a subject's chest, typically sub dermally.

In any embodiment, the controller and power unit (and optionally the electronic circuitry) are contained within a single implantable housing.

In any embodiment, the system comprises an access sheath having a lumen configured for percutaneous delivery of the blood pumping device to the left ventricle.

In any embodiment, the system comprises a delivery shaft for the blood pumping device to advance the blood pumping device through the lumen of the access sheath.

In any embodiment, the delivery shaft and blood pumping device are configured for detachable coupling together. This allows the blood pumping device to be attached to the delivery shaft, advancement of the shaft through the access sheath to deliver the device, and then detachment of the delivery shaft from the blood pumping device.

In any embodiment, the system comprises a handle attached to a proximal end of the access sheath, typically via a first haemostasis valve.

In any embodiment, a proximal end of the delivery shaft is attached to the handle, typically via a second haemostasis valve.

In any embodiment, the handle comprises a first actuator to adjust the axial position of the delivery shaft relative to the access sheath.

In any embodiment, the handle comprises a second actuator to rotate the delivery shaft relative to the access sheath.

In another aspect, the invention provides a method of treating a disease comprising the steps of:
delivering an implantable medical device inside a subject, and
operably connecting the implantable medical device to a remote motor to mechanically power the implantable medical device via a drive shaft configured to drive the implantable medical device to treat the patient.

In any embodiment, the drive transferred by the drive shaft from the motor to the implantable medical device is torque, rotary motion, translational movement or any combination thereof.

In any embodiment, the drive shaft comprises a flexible shaft.

In any embodiment, the method comprises the step of controlling output parameters from the implantable medical device in accordance with the treatment required.

In any embodiment, the method comprises the step of controlling the amount of drive required to drive the implantable medical device.

In any embodiment, the method is a method of treating heart failure or of delivering a drug to a subject with the implantable medical device.

In another aspect, the invention provides a method, for example a method of treating or preventing heart failure, for example treating a subject with a heart condition such as HFpEF, comprising the steps of:
delivering a blood pumping device inside a subject's heart and positioning the blood pumping device to draw blood from the left atrium into the left ventricle, and
operably connecting the blood pumping device to a remote motor to mechanically power the blood pumping device via a drive shaft configured to drive the blood pumping device to treat the patient.

In any embodiment, the drive transferred by the drive shaft from the motor to the implantable medical device is torque, rotary motion, translational movement or any combination thereof.

In any embodiment, the drive shaft comprises a flexible shaft.

In any embodiment, the method comprises activating and deactivating the motor and/or the blood pumping device in a pattern synergistic with the cardiac cycle of the subject comprising activation during ventricular diastole and deactivation during ventricular systole.

In any embodiment, the method comprises optionally anchoring the blood pumping device to the subject's heart.

In any embodiment, the method is a method of treating heart failure with preserved ejection fraction (HFpEF).

In any embodiment, the method is a method of relieving or preventing secondary pulmonary hypertension.

In any embodiment, the method comprises:
sensing with a sensor a heart rate of the subject; and
activating and deactivating the motor and/or the blood pumping device in a pattern (e.g., at a frequency) synchronous with the sensed heart rate of the subject.

In any embodiment, the method comprises modulating the frequency of activation and deactivation of the motor and hence the blood pumping device according to changes in the sensed heart rate over time.

In any embodiment, the step of modulating the frequency of activation and deactivation of the motor comprises modulating the frequency of the voltage duty cycle to the motor.

In any embodiment, the method comprises:
sensing with a sensor a blood pressure parameter the subject's heart, typically a blood pressure parameter of a left side of the heart, preferably a left atrial or left ventricular blood pressure parameter; and
during activation of the blood pumping device, activating the motor and/or the blood pumping device (e.g., modulating the speed of rotation of an impeller of the blood pumping device) at a pumping speed appropriate to the sensed blood pressure parameter.

In any embodiment, the method comprises modulating the pumping speed of the blood pumping device according to changes in the sensed blood pressure parameter over time.

In any embodiment, the step of modulating the pumping speed of the blood pumping device comprises modulating the amplitude of the voltage supply to the motor and hence the drive applied to the blood pumping device.

In any embodiment, the method comprises activating the impeller of the blood pumping device at an impeller rotational speed of 5,000 rpm to 50,000 rpm.

In any embodiment, the method comprises activating the impeller of the blood pumping device at an impeller rotational speed of 10,000 rpm to 40,000 rpm.

In any embodiment, the method comprises reducing the pressure in the left ventricle by at least 5-10 mmHg during ventricular diastole.

In any embodiment, the method comprises delivering the blood pumping device to the left ventricle percutaneously.

In any embodiment, the blood pumping device is percutaneously delivered to the left ventricle via a femoral vein --> iliac vein --> inferior vena cava ---> right atrium--> interatrial septum ---> left atrium ---> left ventricle

In any embodiment, the method comprises implanting a sensor in communication with, or into, the heart of the subject. Typically, the sensor is a blood pressure parameter sensor. In one embodiment, the sensor is a heart pacing device.

In any embodiment, the method comprises:
sensing, by the heart rate sensor, the heart rate of the subject; and
activating and deactivating the blood pumping device based on sensed heart rate data received from the heart rate sensor.

In any embodiment, the method comprises:
sensing, by the blood pressure parameter sensor, a blood pressure parameter of the subject's heart; and
modulating the pumping speed of the blood pumping device based on sensed blood pressure parameter data received from the blood pressure sensor.

In any embodiment, the method comprises:
advancing an access sheath percutaneously along a guidewire so that a distal end of the access sheath is disposed within the left ventricle;
advancing the blood pumping device along a lumen of the access sheath and beyond a distal end of the access sheath;
optionally deploying an anchoring module to anchor the blood pumping device to a wall of the left ventricle.

In any embodiment, the method comprises a step of the anchoring module self-deploying.

In any embodiment, the method comprises delivering an anchoring module to the left ventricle and coupling the blood pumping device to the anchoring module.

In any embodiment, the method comprises deploying the anchoring module to anchor the anchoring module in the heart and coupling the blood pressure device to the anchoring module.

The system of the invention facilitates the elimination of motors from mechanical implantable medical devices so that the size of the medical device can be reduced to facilitate easier implantation of the via the vasculature. Furthermore, by facilitating the selectively remote and separate positioning of motors used to drive implantable medical devices away from the medical device through the use of a flexible shaft which operably connects the separate or remote motor to the medical device to drive the medical device, sensitive tissue such as heart tissue is not exposed to excessive heat generated by the motors . The remote motor, whether incorporated into the controller device or separate from the controller device, is easily accessible for servicing or replacement if required. In addition, the separate remote motor can be selectively positioned externally on a user to help prevent overheating thus improving motor performance. Due to its flexibility, the flexible shaft can also easily navigate turns and obstructions in the heart, vasculature and in or around other organs as required en route from implantable medical devices to the motor. The system of the invention finds particular utility in systems and methods of treating heart failure with preserved ejection fraction (HFpEF) through the use of blood pumping devices, and a method of relieving or preventing secondary pulmonary hypertension. However, as will be appreciated by those skilled in the art, the system can be employed in other systems and methods employing implantable medical devices, both temporary and permanent, particularly mechanical implantable devices such as other pump devices, such as ballon pumps (intra-aortic balloon pump therapy) and drug delivery devices.

### Brief Description of the Figures

The invention will now be described having regard to the accompanying drawings in which:
**FIG. 1** is a perspective view of an exemplary implantable medical device in the form of a blood pumping device which can form part of a drive system of the invention configured to apply torque or rotational velocity to the blood pumping device to treat heart failure with preserved ejection fraction (HFpEF) with the housing of the blood pumping device being transparent to show the interior components of the device;
**FIG.2** is an alternative perspective view of a blood pumping device of Figure 1;
**FIG. 3** is a further alternative view of a blood pumping device of Figure 1;
**FIG. 4** is a perspective view of a blood pumping device of Figure 1 with a non-transparent housing;
**FIG. 5** is a perspective view from above and one side of a drive transfer system of the invention for remotely driving the blood pumping device of Figures 1 to 4 made up of a remote motor, a controller device and a flexible drive shaft for operably connecting the blood pumping device (omitted for clarity) and the remote motor;
**FIG. 6** is an exploded view from above and one side of the drive transfer system of Figure 5;
**FIG. 7** is an illustration of a human heart with a blood pumping device of a system of the invention implanted in the left ventricle and anchored in the left ventricle with the inlet facing the mitral valve to pull blood into the LV from the RA when activated during diastole showing the controller and motor located remotely and externally of the heart and the motor connected to the blood pumping device by a flexible drive shaft;
**FIG. 8** shows a block diagram of one embodiment of the main components of the system of the invention deployed for use in the heart, and
**FIG. 9** is an illustration of the system of the invention with the blood pumping device mounted at the LV of a patient's heart and the motor generating torque/rotation velocity disposed remote from the heart and operably connected to the blood pumping device via a flexible power transfer shaft.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

Additionally, the terms "treatment" or "treating" refers to an intervention (e.g., the use of a blood pumping device to assist systole of the left ventricle) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Implantable medical device" means any device which is placed below the surface of the body to provide support to organs or tissues or to deliver medication or other substances to the body. The implantable medical device can be a mechanical device such as pumping devices including blood pumping devices, ballon pumps (intra-aortic balloon pump therapy) and drug delivery devices.

"Blood pumping device" means a device configured for implantation in a chamber of the heart, particularly the left ventricle or between the left ventricle and the left atrium, that upon activation can draw blood into the left ventricle from the left atrium through the mitral valve during diastole, thus assisting the left ventricle fill with blood. The device generally comprises an impeller. The impeller typically comprises a rotor with vanes disposed within a housing with a blood inlet and a blood outlet. The housing is generally tubular and elongated wherein the blood inlet is disposed at one end or the middle of the housing and the blood outlet is disposed at one end of the housing. The impeller is mounted for rotation within the housing with the vanes disposed towards the blood outlet of the housing. In use, the device is typically anchored to a wall of the left ventricle such that the blood inlet of the housing is positioned closer to the mitral valve than the blood outlet of the housing. The housing typically has a longitudinal axis that in use is directed towards the mitral valve. The device may also include the controller and a sensor. The device may include or be operatively coupled to an inductive charging apparatus.

"Drive shaft" is a shaft that can transmit drive in the form of torque, rotary motion (rotational velocity) or translational movement (such as via an actuator at the shaft or a piston) to an implantable medical device from a remote motor. The drive shaft can be a "flexible shaft" or "flexible drive shaft" which is a drive shaft that can transmit drive such as torque, rotary motion such as rotational velocity, translational movement, or any combination thereof, to an implantable medical device from a motor and is in the form of a flexible cable of any desired length so that it can be routed over, under and around obstacles. In the systems of the invention, the drive shaft can operably connect a remote motor to an implanted medical device such as a blood pumping device. In one form, a flexible shaft can be formed from tightly wound helical springs wrapped around a central wire. The flexible shaft can be attached to and detached from a motor or a controller device incorporating the motor during implantation and removal of the blood pumping device. The flexible shaft can be surrounded by a catheter to prevent the cable from contacting internal tissue. The flexible shaft can also be referred to as a torque cable.

"Output parameters" as applied to the implantable medical device means the treatment(s) performed by the i9mplnatable medical device. The output parameters of the blood pumping device include the frequency of activation of the device and the pump flow pressure (e.g., impeller rotational speed during activation. It will be appreciated that during phases of deactivation of the blood suction device that the impeller rotation may be maintained but at a rotational speed that is significant reduced compared with an activation phase. Thus, for example the impeller may rotate at a rotational speed of 50% or less than the activation phase rotational speed.

"Activation during ventricular diastole and deactivation during ventricular systole" mans that the blood pumping device is activated during at least a part of ventricular diastole (usually at least 50%, 60%, 70%, 80%, 90% or 100% of ventricular diastole) and the blood pumping device is deactivated during ventricular systole. For example, the blood pumping device may be deactivated when system detects closure of the aortic valve (during ventricular systole) and may be activated when the system the opening of the aortic valve (during ventricular filling and subsequent atrial contraction). The blood pumping device may be activated and deactivated at a frequency based on the frequency of the heartbeat detected by, for example, a heart pacing sensor.

"Anchor" or "anchor assembly" means an element configured for engaging tissue, for example a wall, septum or apex of the left ventricle or left atrium of the heart. The anchoring assembly may comprise one, two, three or more armlike anchors. The anchoring assembly comprises anchors that are configured to be deployed and embedded in the tissue of the wall of the left ventricle or left atrium to ensure a secure attachment of the blood suction device. Anchoring is usually at a minimum of two points in the wall. The anchors are adapted to withstand repeated loads generated by both the heart suction device, as well as loads generated by the tissue in which they are embedded. In addition, the anchors are designed to minimise damage to the surrounding tissue, through designs that minimise the damage caused through penetration of the anchor through muscle tissue, and any subsequent damage to the tissue generated by repeated and continuous contraction cycles. The anchor may be formed from any suitable structure. Anchors are selected according to tissue type and the condition of the tissue. In one embodiment, the anchor is encapsulated with an elastic polymer membrane that expands circumferentially when the anchoring assembly is compressed at deployment configuration.

In one embodiment, the coupling between at least one of the anchors and the blood suction device comprises a magnet or magnetisable element. This facilitates coupling of the anchor and the blood suction device at a target location in-vivo. In one embodiment, the anchors are configured for detachable coupling with the heart suction device. This allows one or both anchors to be delivered to the target location separately from the heart suction device. In one embodiment, the device is delivered in two parts, a first part comprising a first anchor, and a second part comprising the heart suction device coupled to the second anchor.

The system of the invention may include an inductive charging apparatus. "Inductive charging apparatus" means an apparatus for charging the system, or any device of the system, wirelessly by using any one of electromagnetic field, wireless radio waves or magnetic resonance charging to transfer energy to charge the device. For example, the blood pumping device may comprise an inductive charging apparatus.

The system of the invention to treat HFpEF is designed to deliver clinically effective functional support to the left ventricle of the heart during the filling phase (diastole). The system increases the flow of blood into the left ventricle of the heart during diastole in which it is implanted and then is deactivated during systole. In one embodiment, the blood pumping device is deactivated during all of ventricular systole. The controller is programmable such that the system can act as an auto-adaptive system that responds to physiologic cues. The system can continuously adapt to specific user requirements. The blood suction device is configured by means of the controller to activate in a pattern synergistic to the natural cycle of the heart to, for example, activate the blood pumping device during diastole and deactivate the blood suction device during systole. Thus, as the heart rate of a subject increases, the frequency of activation and deactivation of the blood suction device changes in sync with the heart rate based on signals received from the sensor. In addition, the controller device can modify the impeller rotation speed based on physiological cues received from the sensor, for example increase the impeller rotational speed during periods of elevated heartbeat and reduce the impeller rotational speed during periods of reduced heart rate. The controller device may comprise a computation device configured to receive data from the sensor, compare the stored with stored reference data, and modify the output parameters of the blood pumping device based on the comparison.

The controller device may be configured for use outside the body. Thus, where the system is for treating HFpEF, the system may include a control lead that operatively couples the heart pumping device implanted within the heart with the controller located outside the body. The control lead may extend through a wall of the heart and through the chest to the external controller. In another embodiment, the control lead may extend percutaneously from the left ventricle through part of the vasculature and out of the body at a suitable location. The controller may be configured to be wearable by a subject. The controller may be connected to a power source, for example a battery. In another embodiment, the controller may be configured for implantation within the heart, as part of the blood suction device, and be configured to receive data from the sensor.

The sensor may be part of the system of the invention, or the system of the invention may be configured for use with a separate sensor. For example, the system may include a sensor configured for implantation in the left ventricle of the heart where it is operatively connected or coupled to the blood pumping device. This may be a wire/sensor element configured to extend into contact with a wall of the heart for sensing a parameter of heart contraction/relaxation or configured to contact blood in the left ventricle or another chamber to detect a parameter of the blood (for example pressure or flow rate).

In another embodiment, the sensor is configured for implantation on an external wall of the heart and operatively coupled to the controller, which may be mounted externally of the subject's body.

In any embodiment, the system comprises a graphical user interface which may form part of the controller. The controller may be configured to display on GUI data relating to the functioning of the system, for example heart rate, blood pressure, frequency of activation of the impeller, rotational speed of the impeller.

In any embodiment, the sensor is configured to detect ejection fraction of the left ventricle. In any embodiment, the controller is configured to compare the detected ejection fraction of the left ventricle with one or more ejection fraction reference values and modify the output parameters of the blood suction device based on the comparison. In any embodiment, the controller is configured to switch off the blood suction device for a period of time when the ejection fraction is detected to be reduced. In any embodiment, the controller is configured to resume operation of the blood suction device when the ejection fraction is detected to be preserved.

The system may further include telemetric components for transmitting and receiving signals relating to the activity of target tissue or organs such as the left ventricle, heart or the activity of the system. Telemetric components may include wireless medical telemetric elements using radio frequency to relay data such as pulse, heart rate, and electrical activity of the heart to the controller.

The controller may be configured to control the operation of the implantable medical device such as the blood pumping device either through automatic execution of program instructions in memory and/or upon receiving an external input from a user. The memory component of the system may include ROM and RAM memory. The controller may take the form of a microprocessor.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

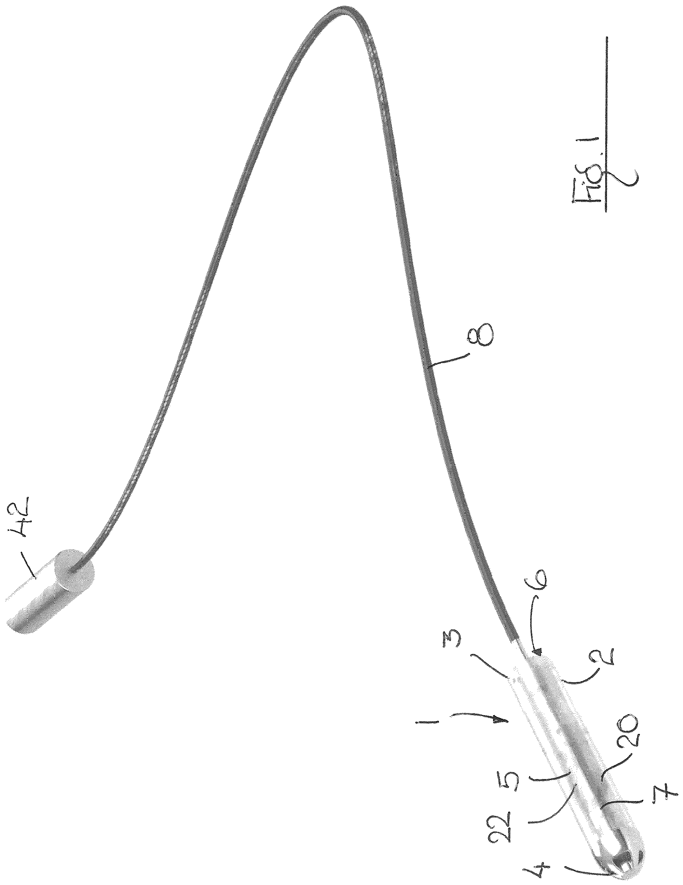

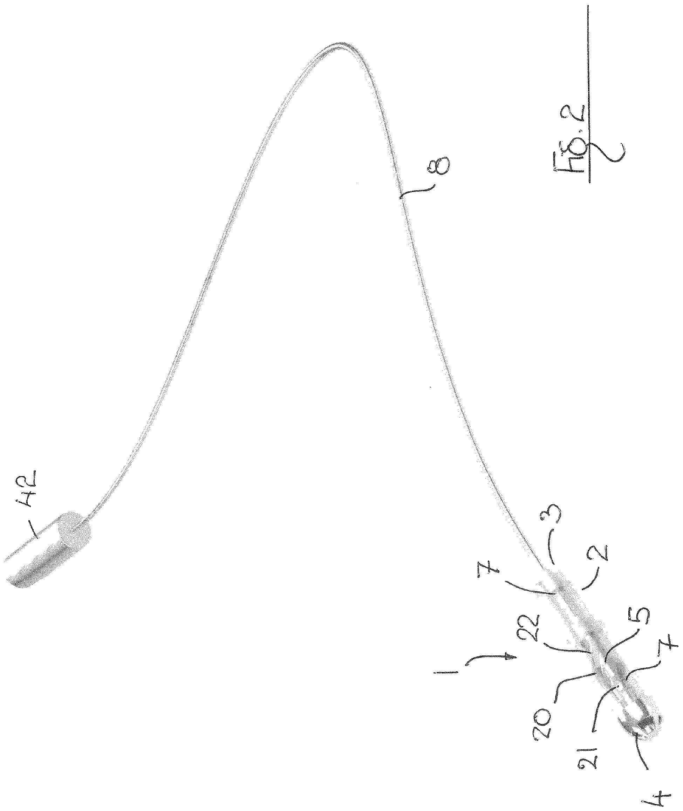

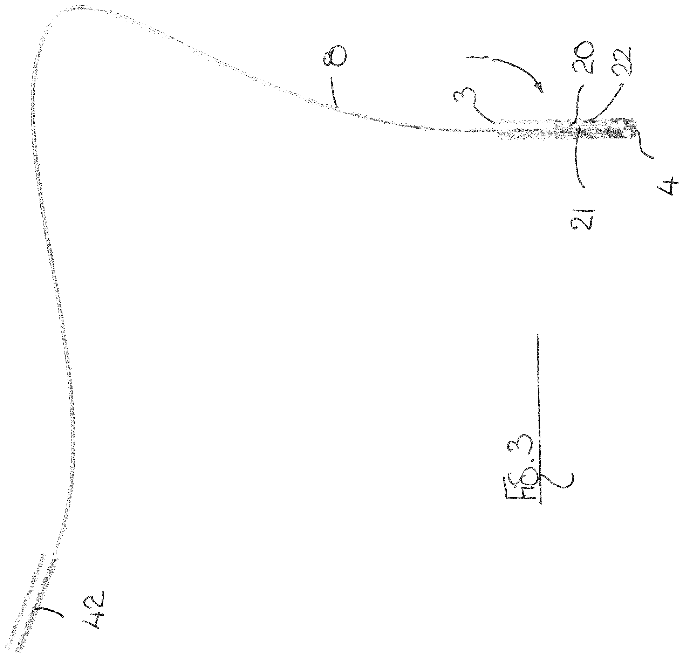

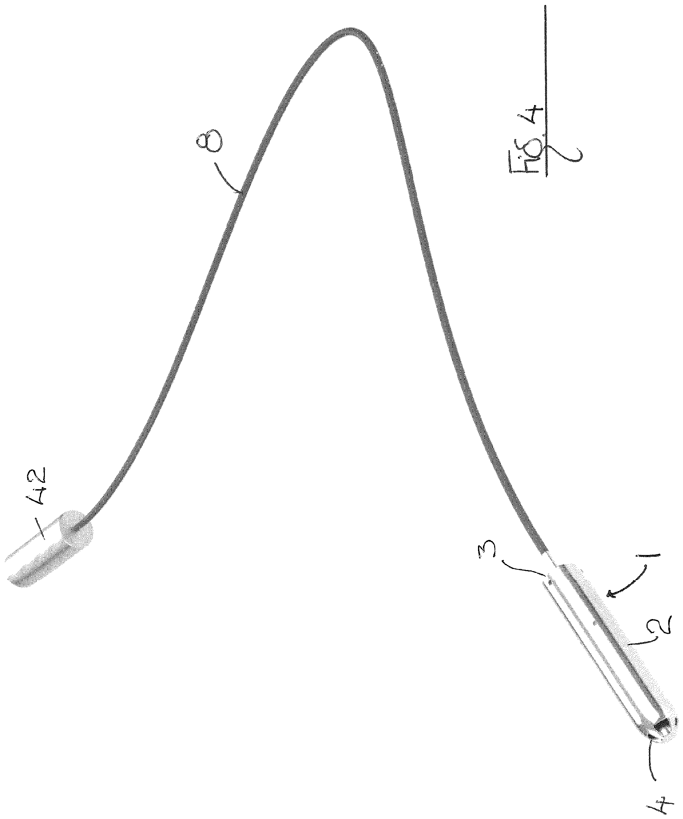

Referring to Figures 1 to 4, there is illustrated an exemplary implantable medical device in the form of a blood pumping device forming part of a system of the invention and indicated generally the reference numeral 1. However, as indicated above, for the purposes of the invention, the implantable medical device can be any device which is placed below the surface of the body to provide support to organs or tissues or to deliver medication or other substances to the body and the blood pumping device is only an example of such devices. Accordingly, the invention should not be construed as being limited to blood pumping devices for cardiac or other purposes.

The device 1 comprises an external tubular housing 2 with blood inlets 3 at one end, a blood outlet 4, and internal lumen 6 extending between the inlet 3 and outlet 4. The housing can have a length of 30 mm and a diameter of 7.6 mm. A rotor 5 is mounted on spaced apart bearings 7 and extends axially through the internal lumen 6 for rotation within the lumen. A flexible drive shaft 8 is operably connected to a proximal end of the rotor 5 adjacent the blood inlet 3 and in use functions to rotate the rotor 5 during activation of the blood pumping device. The flexible shaft 8 can be connected to the rotor via fittings (not shown) between the flexible shaft 8 and the rotor 5. As shall be explained more fully below, the flexible shaft 8 is configured to drive the rotor 5 by transmitting torque or rotational motion/velocity from a power source/motor 42 which is remote or displaced from the blood pumping device 1 e.g. a motor 42 mounted external to the body or implanted elsewhere on the body outside the heart.

An impeller device 20 is mounted to a distal end of the rotor 5 and can comprise a tubular hub 21 with a diameter of 1.665 mm and elongated swept vanes 22 (e.g. 2) attached to opposite sides of the hub in a symmetrical manner. Each vane has an axial length of 13 mm and a radial width of 4.995 mm and sweeps around the hub along its length from a distal end to a proximal end at an angle of about 100° (sweep angle). The clearance between each vane and the surrounding external housing is 0.5 mm.

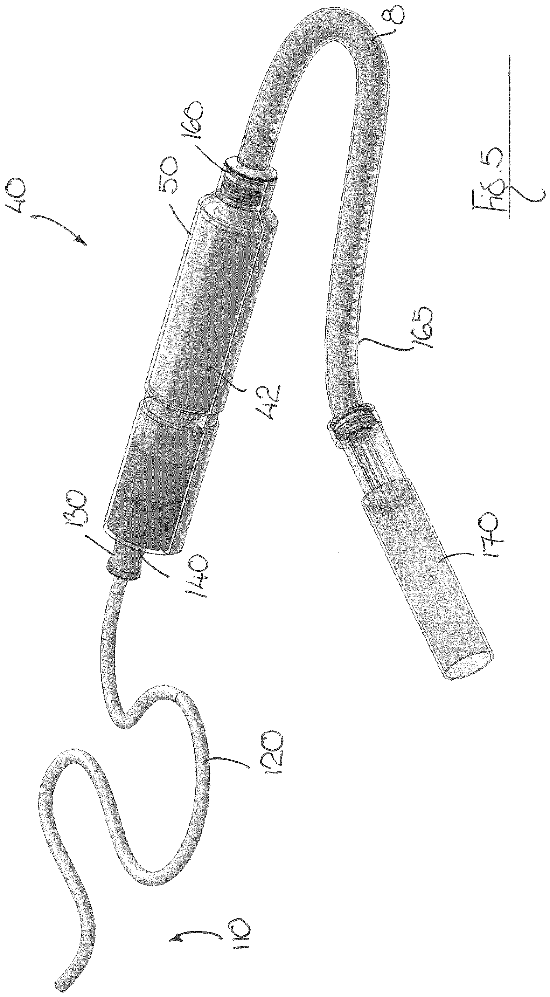

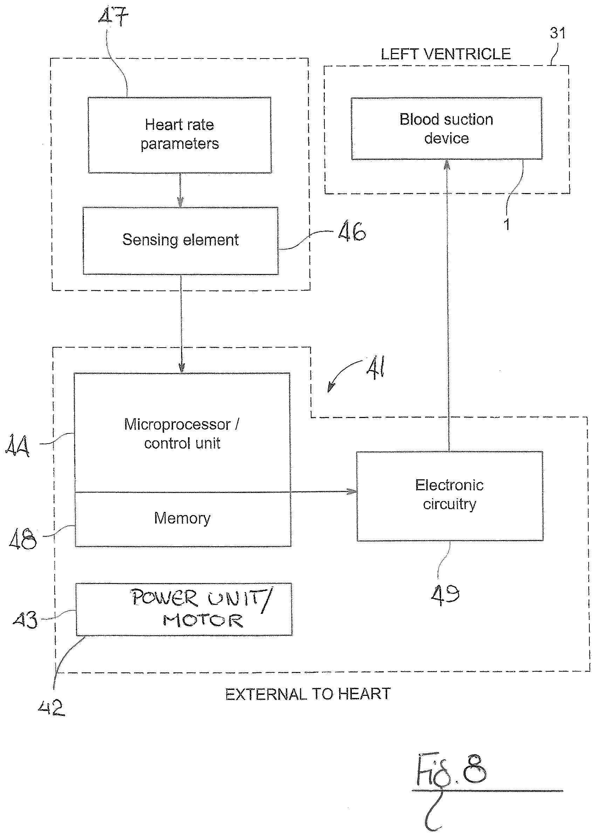

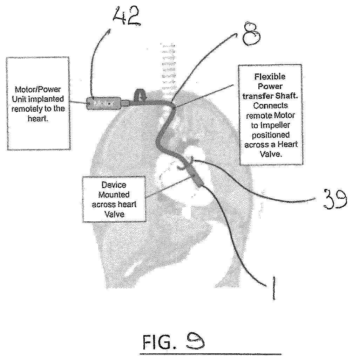

Figures 5 to 9 show a drive transfer system of the invention generally indicated by the reference numeral 40 to apply drive which in the present embodiment of the invention is torque or rotational motion to an implantable medical device in the form of the exemplary blood pumping device 1 of Figures 1 to 4 to treat cardiac disease such as heart failure with preserved ejection fraction (HFpEF) with Figure 8 being a block diagram of the main components of the system 40 and the location the components of the system relative to the heart. However, as indicated above, the system of the invention should not be construed as being limited to systems comprising implantable medical devices in the form of blood pumping devices as in other embodiments the system can include other implantable medical devices such as drug delivery devices and the like.

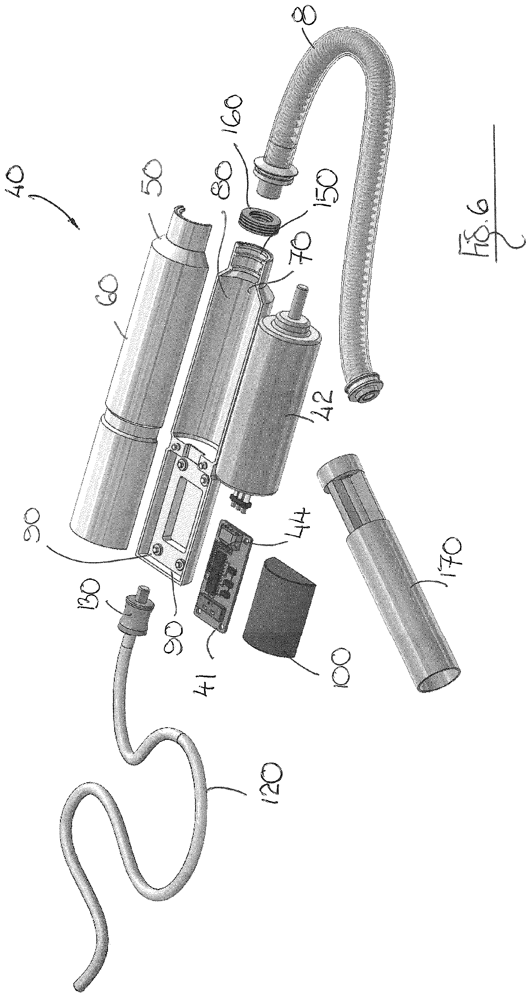

As shown in Figures 5 and 6, the drive transfer system 40 is made up of a motor 42 separate and remote from the blood pumping device 1. The motor 42 can be housed in a motor casing or housing 50 made up of a top cover 60 and a bottom cover 70 which can be attached to and detached from each other to provide easy access to a motor chamber 80 for the motor 42.

The motor housing 50 also defines an internal controller device chamber 90 for containing a controller device 41 which can therefore also be remote from the blood pumping device 1. A controller/processing device 44 can also be provided within the controller device 41. In other embodiments of the invention, the controller device 41 can be separately housed in its own remote housing if desired. The controller device chamber 90 can also accommodate a power unit 100 such as a battery pack 100 (which can be rechargeable e.g. wirelessly or via a charging cable) if desired to power the motor 42 or to act as a supplementary/back-up/mobile power source to an external power supply indicated by the reference numeral 110 for the motor 42. The external power supply 110 is connected to the motor 42 via a power supply cable 120 with an input 130 attachable to and detachable from a first end of the motor housing 50 at an input socket 140. The controller device 41 can be configured to modify the output parameters of the motor 42 in accordance with the treatment required.

The motor housing 50 can be external to the subject's body or implanted within the body (e.g. sub-dermally). Due to its remote positioning, the motor 42 can be replaced as required without requiring access an implanted blood pumping device 1.

A drive shaft 8, which in the present embodiment can be a flexible drive shaft 8, is operably connected between the remote motor 42 and the blood pumping device 1 to drive the blood pumping device 1. In the present embodiment, the flexible drive shaft 8 is operably connectable between the motor 42 and an implantable blood pumping device via a shaft connector 150 at a second end of the motor housing 50. The shaft connector 150 can be provided with a fluid sealing package 160 and can be surrounded by a protective catheter 165.

At its opposite end, the drive shaft 8 is operably connectable to the implantable medical device 1 which can be removably mounted in an optional implant casing 170. In the present embodiment, the implant casing 170 is a cylindrical construct for housing and protecting a medical device. However, in other embodiments of the invention, the implant casing 170 can be omitted.

The flexible shaft 8 can be attached to and detached from controller device 41/motor 42 during implantation and removal of the blood pumping device 1. The motor 42 is configured to deliver the desired level of torque and rotational velocity to the blood pumping device 1 and the controller device 41 can specify the amount of torque and rotational velocity to be delivered instantaneously to the blood pumping device 1.

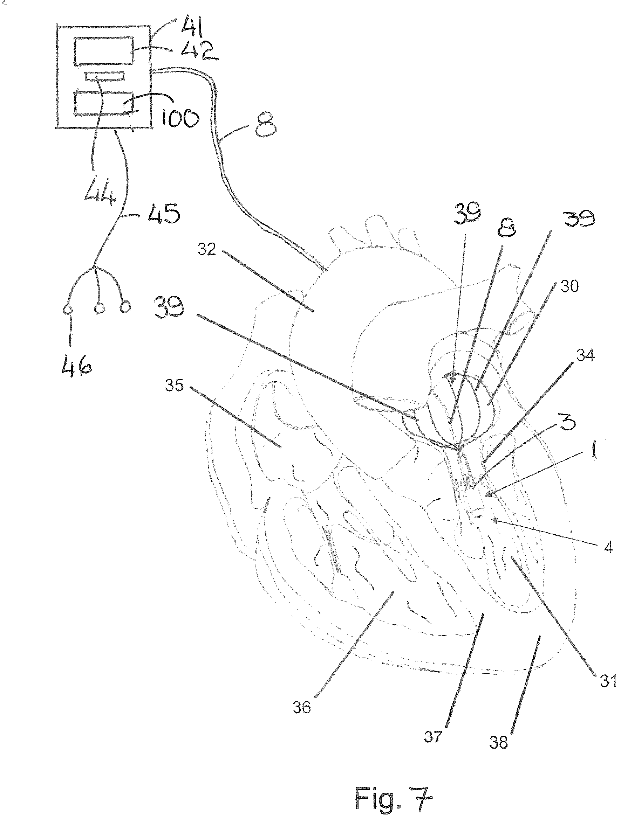

Figure 7 illustrates a human heart including left atrium 30, left ventricle 31, aorta 32 and aortic cusps 33, mitral valve 34, right atrium 35, right ventricle 36, left ventricular septum 37 and left ventricular apex 38. The blood pumping device 1 is located in a sub-valvular position just below the mitral valve 34 and is implanted with an anchoring assembly 39 made up of arms 39 movable between deployed and retracted positions located in the left atrium 30. The device is positioned such that the blood inlet 3 of the device 1 faces towards the mitral valve 34 and left atrium 30 and the blood outlet 4 faces the left ventricle 31. It will be appreciated by those skilled in the art that the blood pumping device 1 and associated anchoring means can be positioned elsewhere in the heart if desired e.g. entirely within the left ventricle 31 and the anchoring assembly 39 may engage the left ventricle 31, pulmonary artery, parts of the mitral valve 34, or a combination of one or more of these. Upon activation of the device during diastole, blood is drawn into the device through the inlet 3 and ejected through the outlet 4 as illustrated by the arrows, which assists the emptying of blood from the left atrium 30 into the left ventricle 31. Once diastole is completed, the device is deactivated as the mitral valve 34 closes and the aortic cusps 33 open, and the left ventricle 31 contracts to pump blood into the aorta 32 unassisted.

As shown in the drawings, in the present embodiment, the drive transfer system 40 of the invention comprises the blood pumping device 1 of Figures 1 to 4 shown implanted in the heart. The rotor 5 of the blood pumping device 1 is connected to the motor 42 of the drive transfer system 40 which is separated and remotely located from the blood pumping device 1 but connectable to the blood pumping device 1 with the flexible power transmission shaft 8. The motor 42 is contained within motor housing 50 which can be external to the subject's body or implanted within the body (e.g. sub-dermally). The motor 42 can be replaced as required without requiring access to the implanted blood pumping device 1. The flexible shaft 8 can be attached to and detached from motor 42/controller device 41 during implantation and removal of the blood pumping device 1. As indicated above, the motor 42 and the power unit 100 can be separate from the controller device 41 or combined with the controller device 41 in a single housing as shown in Figures 5 and 6.

A sensing lead 45 operatively connects the controller device with an ECG sensor 46 mounted to the subject's chest. The ECG array has one or more leads that are placed on the skin of the user. The controller device 41 receives the electrical parameters and activates and deactivates the blood pumping device 1 in a pattern synergistic with the electrical parameters to activate the blood suction device during diastole and deactivate the blood suction device during systole to assist the LV ventricle fill with blood during diastole. The controller device 41 also controls other parameters of the blood suction device based on data received from the sensor, including increasing the impeller rotational speed via the flexible power transmission shaft 8 during diastole during periods of activity (e.g., when the heartbeat rises above a resting heart rate) and lowering the impeller rotational speed via the flexible power transmission shaft 8 during diastole during periods of rest.

As shown particularly in Figure 8, the blood pumping device 1 comprising an impeller 20 is located at the left ventricle 31 of the subject's heart while being anchored optionally in the left atrium 30. A sensor 46 configured to detect a heart rate parameter is located outside of the heart. The sensor 46 may be an ECG sensor comprising ECG leads 45 disposed on the subject's chest as previously described, or another type of heart rate parameter sensor located externally of the subject's heart. The sensor 46 is configured to detect a heart rate parameter 47, in this case left ventricle contraction parameters.

The sensor data output from the sensor 46 is communicated to the controller device 41 by means of either wired or wireless communications. The controller device 41 is configured to continuously assess the time of diastole and systole based on the received sensor data and optimally synchronise the activation and deactivation of the blood suction device 1 into sync with the diastole and systole stages of the cardiac cycle.

The blood pumping device 1 and similar implantable medical devices may be delivered percutaneously via femoral artery/subclavian artery or femoral vein/subclavian vein. The blood pumping device 1 may also be delivered through transapical route, whereby an incision is given in an intercostal space and the device is delivered through the apex.

The flexible shaft 8 may be attached to and detached from implantable medical devices, such as the blood pumping device, during implantation and removal of the medical device.

In use, the sensor 46 detects the heart rate parameters of the heart and sends the sensor data to the controller device 41. The received sensor data is analysed by the controller device 41 according to the instructions stored in a memory 48. The controller device 41 then delivers control signals to the motor42/power unit 100 and to electronic circuitry 49 so as to modify the output parameters of the heart pumping device 1 as appropriate based on the sensor data so as to activate the heart pumping device 1, via the flexible power transmission shaft 8, in a pattern synergistic to the natural contraction cycle of the heart to activate the device during diastole and deactivate the device during systole.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A drive transfer system configured to drive a remote implantable medical device configured to treat disease, the system comprising:
a motor configured to mechanically actuate an implantable medical device:
a controller device communicable with the motor configured to modify the output parameters of the motor in accordance with the treatment required, and
a drive shaft configured to operably connect the motor to the implantable medical device to drive the implantable medical device when the motor is located remotely from the implantable medical device.

2. A system according to Claim 1, wherein the drive transferred by the drive shaft from the motor to the implantable medical device is torque, rotary motion, translational movement or any combination thereof.

3. A system according to Claim 1 or Claim 2, wherein the drive shaft comprises a flexible shaft.

4. A system according to Claim 3, wherein the flexible shaft comprises a flexible cable.

5. A system according to Claim 3 or Claim 4, wherein the flexible shaft is surrounded by a catheter.

6. A system according to any of Claims 1 to 5, wherein the motor is optionally integral with the controller device and the shaft is configured to attach to and detach from the motor and/or the controller device.

7. A system according to any of Claims 1 to 6, wherein the controller device comprises an implantable controller device.

8. A system according to any of Claims 1 to 7 wherein the controller device is configured to control the amount of drive required to drive the implantable medical device.

9. A system according to any of Claims 1 to 8, further comprising a power unit to power the motor.

10. A system according to any of Claims 1 to 9, further comprising an implantable medical device.

11. A system according to Claim 10, wherein the implantable medical device comprises a pump.

12. A system according to any of Claims 1 to 11, wherein the disease is a cardiac disease.

13. A system according to Claim 12, wherein the controller device is configured to modify the output parameters of the implantable medical device or the motor so as to activate and deactivate the implantable medical device or motor.

14. A system according to Claim 12 or Claim 13, further comprising at least one sensor in communication with the controller device to detect one or more parameters associated with the heart, wherein the controller device is configured to modify the output parameters of the implantable medical device or the motor based on the one or more detected parameters received from the at least one sensor and in which the at least one sensor is optionally configured to detect one or more parameters selected from heart rate, closure of the aortic valve, opening of the aortic valve, closure of the mitral valve, and opening of the mitral valve, or a blood pressure parameter of the subject's heart and the controller device is configured to adjust the frequency of activation and deactivation of the implantable medical device or the motor based on the one or more parameters sensed by the sensor.

15. A system according to any of Claims 10 to 14, in which the pump is a blood pumping device comprising:
an external tubular housing;
a blood inlet in the tubular housing,
an oppositely disposed blood outlet in the tubular housing;
an internal lumen extending between the blood inlet and blood outlet, and
an impeller mounted on a rotor within the housing to pump blood from the blood inlet to the blood outlet, wherein the drive shaft is operably connected to the rotor to rotate the rotor during activation of the blood pumping device.
